(19) Europäisches Patentamt
European Patent Office
Office européen des brevets

(11) **EP 1 716 426 B1**

(12) **EUROPÄISCHE PATENTSCHRIFT**

(45) Veröffentlichungstag und Bekanntmachung des Hinweises auf die Patenterteilung:
**13.02.2013 Patentblatt 2013/07**

(21) Anmeldenummer: **05700536.5**

(22) Anmeldetag: **13.01.2005**

(51) Int Cl.:
***G01R 33/28*** *(2006.01)*          ***A61K 49/18*** *(2006.01)*

(86) Internationale Anmeldenummer:
**PCT/DE2005/000023**

(87) Internationale Veröffentlichungsnummer:
**WO 2005/069027 (28.07.2005 Gazette 2005/30)**

(54) **VERFAHREN ZUR HYPERPOLARISATION VON ATOMKERNEN UND VORRICHTUNG ZUR DURCHFÜHRUNG DES VERFAHRENS**

METHOD FOR THE HYPERPOLARISATION OF ATOMIC NUCLEI AND DEVICE FOR CARRYING OUT SAID METHOD

PROCEDE D'HYPERPOLARISATION DE NOYAUX ATOMIQUES ET DISPOSITIF CORRESPONDANT

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **19.01.2004 DE 102004002640**
**12.05.2004 DE 102004023345**

(43) Veröffentlichungstag der Anmeldung:
**02.11.2006 Patentblatt 2006/44**

(73) Patentinhaber: **Forschungszentrum Jülich GmbH**
**52425 Jülich (DE)**

(72) Erfinder:
 • **APPELT, Stephan**
  **52070 Aachen-Soers (DE)**
 • **HÄSING, Friedrich, Wolfgang**
  **52351 Düren (DE)**
 • **D'ORSANEO, Giovanni**
  **52428 Jülich (DE)**
 • **SIELING, Ulrich**
  **41812 Erkelenz (DE)**

(56) Entgegenhaltungen:
WO-A-99/08766     DE-A1- 19 937 566
US-A- 5 860 295     US-A1- 2002 107 439

 • **GOODSON B M: "ADVANCES IN MAGNETIC RESONANCE NUCLEAR MAGNETIC RESONANCE OF LASER-POLARIZED NOBLE GASES IN MOLECULES, MATERIALS, AND ORGANISMS" JOURNAL OF MAGNETIC RESONANCE, ACADEMIC PRESS, ORLANDO, FL, US, Bd. 155, Nr. 2, April 2002 (2002-04), Seiten 157-216, XP001115307 ISSN: 1090-7807**

**Beschreibung**

Die Erfindung betrifft ein Verfahren zur Hyperpolarisation von Atomkernen und eine Vorrichtung zur Durchführung des Verfahrens.

In US 5,860,295 ist die Anreicherung von hyperpolarisiertem $^{129}$Xe beschrieben, enthaltend die Schritte gemäß der Präambel des Anspruchs 1.

Neuere Entwicklungen in der Magnet Resonanz Tomographie (MRT) sowie in der Magnet Resonanz Spektroskopie (NMR) mit polarisierten Edelgasen lassen Anwendungen in der Medizin, in der Physik und in den Materialwissenschaften erwarten. Die Polarisation von Edelgaskernen kann durch optisches Pumpen mit Hilfe von Alkaliatomen erzielt werden, wie der Druckschrift Happer et al., Phys. Rev. A, 29, 3092 (1984) zu entnehmen ist.

Der Begriff des optischen Pumpens umfasst das von Kastler entwickelte Verfahren, durch Lichteinstrahlung in Materie die Besetzungszahlen bestimmter Energiezustände gegenüber dem Gleichgewichtszustand bedeutend zu erhöhen. Durch optisches Pumpen können die relativen Besetzungszahlen von Energieniveaus in Atomen, Ionen, Molekülen und Festkörpern verändert und Ordnungszustände hervorgerufen werden. Die Besetzungsdichte des optisch gepumpten Zustandes weicht deutlich von seiner thermischen Besetzungswahrscheinlichkeit gemäß der Boltzmann-Verteilung ab. Durch optisches Pumpen von Zeeman-Niveaus kann z.B. eine Parallelstellung der magnetischen Momente von Atomen und Atomkernen erreicht werden.

Typischerweise wird in der Praxis das Alkaliatom Rubidium in Anwesenheit eines Edelgases und Stickstoff eingesetzt. Dabei ist es möglich, eine Kernspinpolarisation von z.B. $^{129}$Xe von etwa 20 Prozent zu erreichen. Eine solche Kernspinpolarisation ist ca. 100000 mal größer als die Gleichgewichtspolarisation in klinischen Magnet Resonanz Tomographen bei 1 T und 300 K. Die damit verbundene drastische Steigerung des Signal-Rausch-Verhältnisses erklärt, weshalb in Zukunft neue Anwendungsmöglichkeiten in der Medizin, Wissenschaft und Technik erwartet werden.

Unter Polarisation wird der Grad der Ausrichtung (Ordnung) der Spins von Atomkernen oder Elektronen verstanden. Zum Beispiel bedeutet 100 Prozent Polarisation, dass sämtliche Kerne oder Elektronen in gleicher Weise orientiert sind. Mit der Polarisation von Kernen oder Elektronen ist ein magnetisches Moment verbunden.

Polarisiertes Xenon wird zum Beispiel von einem Menschen inhaliert oder in ihn injiziert. 10 bis 15 Sekunden später sammelt sich das polarisierte Xenon im Gehirn an. Mit Hilfe der Magnetischen Resonanz Tomographie wird die Verteilung des Edelgases im Gehirn festgestellt. Das Ergebnis wird für weitere Analysen genutzt.

Die Wahl des Edelgases hängt jeweils vom Anwendungsfall ab. $^{129}$Xe weist eine große chemische Verschiebung auf. Wird Xenon z.B. auf einer Oberfläche adsorbiert, so verändert sich signifikant seine Resonanzfrequenz. Außerdem löst sich Xenon in lipophilen Flüssigkeiten. Wenn derartige Eigenschaften erwünscht sind, wird Xenon eingesetzt.

Das Edelgas Helium löst sich kaum in Flüssigkeiten. Das Isotop $^3$He wird daher regelmäßig dann verwendet, wenn Hohlräume betroffen sind. Die Lunge eines Menschen stellt ein Beispiel für einen solchen Hohlraum dar.

Einige Edelgase weisen andere wertvolle Eigenschaften als die vorgenannten auf. So besitzen z.B. die Isotope $^{83}$Kr, $^{21}$Ne und $^{131}$Xe ein Quadrupolmoment, welches z.B. für Experimente in der Grundlagenforschung bzw. in der Oberflächenphysik interessant sind. Diese Edelgase sind allerdings sehr teuer, so dass diese für Anwendungen, bei denen größere Mengen verwendet werden, ungeeignet sind.

Aus der Druckschrift Driehuys et al. (Appl. Phys. Lett. (1996). 69, 1668) ist bekannt, Edelgase auf folgende Weise in einem Polarisator zu polarisieren.

Ausgehend von einer Gasversorgung, wird ein Gasstrom, bestehend aus einem Gemisch von $^{129}$Xe, $^4$He und $N_2$ in einem Rb-Behälter mit Rb-Dampf angereichert und durch eine Pumpzelle geleitet. Mit Hilfe eines Lasers wird zirkular polarisiertes Licht bereitgestellt, also Licht, bei dem der Drehimpuls bzw. der Spin der Photonen alle in die gleiche Richtung zeigen. In der Pumpzelle werden die Rb-Atome als pumpbare Spezies mit dem Laserstrahl ($\lambda \sim$ 795 nm, Rb D1-Linie) longitudinal zu einem Magnetfeld optisch gepumpt und so die Elektronenspins der Rb-Atome polarisiert. Dabei wird der Drehimpuls der Photonen auf freie Elektronen von Alkaliatomen übertragen. Die Spins der Elektronen der Alkaliatome weisen somit eine große Abweichung vom thermischen Gleichgewicht auf. Die Alkaliatome sind folglich polarisiert. Durch einen Stoß eines Alkaliatoms mit einem Edelgasatom wird die Polarisation des Elektronenspins vom Alkaliatom auf das Edelgasatom übertragen. Es entsteht so polarisiertes Edelgas. Die durch das optische Pumpen von Alikaliatomen erzeugte Polarisation des Elektronenspins der Alkaliatome wird also durch Spinaustausch vom Alkali-Elektron auf den Kernspin der Edelgase übertragen, wie erstmals von Bouchiat am Rb/$^3$He-System gezeigt wurde.

Aus Appelt et al. (S. Appelt, A. Ben-Amar Baranga, C. J. Erickson, M. V. Romalis, A. R. Young, and W. Happer, Phys. Rev. A (1998), 58, 1412) ist zur Theorie von Zweikörperstößer bekannt, zwischen einem Paar von Alkalimetall-Atomen einen Spinaustausch herbeizuführen.

Aus WO 99/08766 ist bekannt, neben einem ersten optisch pumpbaren Alkalimetall ein Hilfs-Alkalimetall als nicht optisch pumpbare Spezies einzusetzen. Die optisch pumpbare Spezies überträgt dabei die Elektronenspinpolarisation auf die nicht optisch pumpbare Spezies, wodurch effektiv eine Erhöhung des Polarisationsgrades des Edelgases eintritt.

Alkaliatome werden eingesetzt, da diese über ein großes optisches Dipolmoment verfügen, welches mit dem Licht wechselwirkt. Ferner weisen Alkaliatome jeweils ein freies Elektron auf, so dass keine nachteilhaften Wechselwirkungen zwischen zwei und mehr Elektronen pro Atom auftreten können.

Cäsium wäre ebenfalls ein gut geeignetes Alkaliatom, welches gegenüber Rubidium zur Erzielung vorgenannter Wirkungen überlegen ist. Es stehen jedoch zur Zeit keine Laser mit genügend hoher Leistung zur Verfügung, wie sie für die Polarisation von Xenon mittels Cäsium benötigt würden.

Um bei der Verwendung von breitbandigen Hochleistungs-Halbleiterlasern möglichst viele Photonen nutzen zu können, wird beim optischen Pumpen von Edelgasen bei Drücken von mehreren Atmosphären gearbeitet. Dabei unterscheidet sich das optische Pumpen von Alkalimetall-Atomen je nach Art des zu polarisierenden Edelgases.

Zur Polarisation von $^{129}$Xe wird ein Gasgemisch unter einem Druck von etwa, 700 bis 1000 kPa (7 bis 10 bar) durch eine zylindrische Glaszelle kontinuierlich oder halbkontinuierlich durchgeleitet. Das Gasgemisch besteht zu 94 Prozent aus $^{4}$He, zu 5 % aus Stickstoff und zu 1 % aus Xenon. Die typische Fließgeschwindigkeit des Gasgemisches beträgt 1 cm je Sekunde.

Im Falle der Polarisation von $^{3}$He wird der nötige Druck im Polarisator durch das $^{3}$He selbst erzeugt, da die Elektronenspinrelaxationsrate von Rb-$^{3}$He-Stößen klein ist. Beim Spinaustauschpumpen von Rb-$^{129}$Xe ist dies nicht der Fall, weshalb der Druck durch ein zusätzliches Puffergas wie $^{4}$He erzeugt wird. Aus den unterschiedlichen Relaxations- und Spinaustauschraten ergeben sich unterschiedliche Anforderungen an die Polarisatoren.

So liegen bei $^{3}$He die Kernspin-Polarisations-Aufbauzeiten im Bereich von Stunden. Da aber auch die Rubidium-Spin-Zerstörungsrate für Rubidium-$^{3}$He-Stöße relativ gering ist, kann hier bei hohen $^{3}$He-Drücken gearbeitet werden (> 500 kPa (5 bar)).

Für $^{129}$Xe hingegen liegen die Kernspin-Polarisations-Aufbauzeiten aufgrund des größeren Spinaustausch-Wirkungsquerschnittes zwischen 20 bis 40 Sekunden. Aufgrund der sehr großen Rubidium-Elektronenspinrelaxationsrate für Rubidium-Xenon-Stöße darf beim optischen Spinaustauschpumpen der Xe-non-Partialdruck nur weniger als 10 kPa (100 mbar) betragen, damit eine genügend hohe Rubidium-Polarisation aufrecht erhalten werden kann. Deshalb wird in solchen Polarisatoren $^{4}$He zur Linienverbreiterung als Puffergas eingesetzt.

Die Polarisatoren können als Fluss-Polarisatoren, z.B. zur Polarisation von $^{129}$Xe oder als Polarisatoren mit einer abgeschlossenen Probenzelle für z.B. $^{3}$He ausgestaltet sein.

In einem Durchflusspolarisator durchströmt das Gasgemisch zunächst ein Gefäß, nachfolgend "Vorratsgefäß" genannt, in dem sich eine gewisse Menge Rb befindet. Das Vorratsgefäß mit dem darin befindlichen Rubidium wird zusammen mit der sich anschließenden Glaszelle auf ca. 100 bis 150 Grad Celsius erwärmt. Durch Bereitstellung dieser Temperaturen wird das Rubidium verdampft. Die Konzentration der verdampften Rubidium-Atome in der Gasphase wird durch die Temperatur im Vorratsgefäß bestimmt. Der Gasstrom transportiert die verdampften Rubidium-Atome von dem Vorratsgefäß z.B. in eine zylindrische Probenzelle. Ein leistungsstarker, zirkular polarisiertes Licht bereitstellender Laser mit ca. 100 Watt Leistung im kontinuierlichen Betrieb durchstrahlt die Probenzelle axial, das heißt in Flussrichtung und pumpt optisch die Rubidium-Atome in einen hochpolarisierten Zustand. Die Wellenlänge des Lasers muss dabei auf die optische Absorptionslinie der Rubidium-Atome (D1-Linie) abgestimmt sein.

Mit anderen Worten: Um die Polarisation vom Licht auf ein Alkaliatom optimal zu übertragen, muss die Frequenz des Lichts mit der Resonanzfrequenz des optischen Übergangs übereinstimmen.

Die Probenzelle befindet sich in einem statischen magnetischen Feld $B_0$ von etwa 1mT (10 Gauss), das von Spulen, insbesondere einem sogenannten Helmholtzspulenpaar, erzeugt wird. Die Richtung des magnetischen Feldes verläuft parallel zur Zylinderachse der Probenzelle bzw. parallel zur Strahlrichtung des Lasers. Das Magnetfeld dient der Führung der polarisierten Atome. Die durch das Licht des Lasers optisch hochpolarisierten Rubidium-Atome kollidieren in der Glaszelle unter anderem mit den Xenon-Atomen und geben ihre Polarisation an die Xenon-Atome ab.

Am Ausgang der Probenzelle scheidet sich das Rubidium aufgrund des hohen Schmelzpunkts im Vergleich zu den Schmelzpunkten der übrigen Gase an der Wand ab. Das polarisierte Xenon bzw. das Restgasgemisch wird von der Probenzelle in eine Ausfriereinheit weitergeleitet. Diese besteht aus einem Glaskolben, dessen Ende in flüssigen Stickstoff getaucht ist. Der Glaskolben befindet sich ferner in einem Magnetfeld mit einer Stärke von > 0.1 T (1000 Gauss). Das hochpolarisierte Xenon-Gas scheidet sich an der inneren Glaswand der Ausfriereinheit als Eis ab.

Am Auslaß der Ausfriereinheit wird das restliche Gas ($^{4}$He und $N_2$) in der Regel über ein Nadelventil geleitet und schließlich abgelassen. Die Flussgeschwindigkeit in der gesamten Anordnung kann über das Nadelventil gesteuert und mit einem Messgerät gemessen werden.

Steigt die Flussgeschwindigkeit zu sehr an, so verbleibt keine Zeit zur Übertragung der Polarisation von den RubidiumAtomen auf die Xenon-Atome. Es wird also nur eine geringe Polarisation erzielt. Ist die Flussgeschwindigkeit zu niedrig, so verstreicht zuviel Zeit, bis die gewünschte Menge an hochpolarisiertem Xenon eingefroren ist. Durch Relaxation im Xe-Eis nimmt die Polarisation der Xenon-Atome nämlich wieder ab. Die Relaxation der Xenon-Atome wird durch das Einfrieren sowie durch ein starkes Magnetfeld, welchem die Ausfriereinheit ausgesetzt ist, stark verlangsamt. Es ist daher erforderlich, nach der Polarisierung das Edelgas Xenon möglichst schnell und verlustfrei einzufrieren. Zwar kann die Relaxation durch das Einfrieren nicht ganz vermieden werden. Es verbleiben jedoch bei 77 K ungefähr 1 bis 2 Stunden Zeit, ehe die Xenon-Polarisation so stark abgenommen hat, dass eine weitere Verwendung des anfangs hochpolarisierten Gases nicht mehr möglich ist.

Ein Polarisator der vorgenannten Art weist stets Verbindungsstellen auf. Verbindungsstellen sind solche, bei denen wenigstens zwei Leitungen, durch die polarisiertes Gas geleitet wird, miteinander verbunden sind. Die Leitungen bestehen in der Regel aus Glas. Die Verbindung wird durch ein Verbindungselement wie z.B. Flansche hergestellt.

Um ein einzelnes freies Alkaliatom zu polarisieren, ist eine bestimmte Energie erforderlich. Die erforderliche Energie entspricht der Resonanzfrequenz zur Anhebung des freien Elektrons des Alkaliatoms von einem Grundzustand in einen angeregten Zustand. Um die Energie von einem Laser auf das Alkaliatom optimal zu übertragen, muss die Frequenz des Lichts des Lasers auf die Resonanzfrequenz des Alkaliatoms abgestimmt werden. Einige Laser senden ihr Licht innerhalb eines bestimmten Frequenzspektrums aus. Es handelt sich dabei also nicht um eine einzelne Frequenz, sondern um eine Verteilung von Frequenzen. Das zur Verfügung stehende Spektrum eines Lasers wird durch die sogenannte Linienbreite charakterisiert. Um wirtschaftlich Alkaliatome zu polarisieren, werden breitbandige Halbleiter-Laser vorgesehen, deren Frequenz und Linienbreite auf die Resonanzfrequenz bzw. die optische Linienbreite des Alkaliatoms abgestimmt sind.

Um die Energie von einem Laser auf Alkaliatome besser übertragen zu können, sind während der Polarisation Stoßpartner für die Alkaliatome vorgesehen. Als Stoßpartner dienen insbesondere die $^4$He-Atome. Durch die Wechselwirkung bzw. durch die Stöße mit den Helium-Atomen verbreitert sich die optische Linienbreite eines Alkaliatoms. Je breiter dieses atomare Spektrum ist, desto eher können spektral breite und damit preiswerte Laser eingesetzt werden.

Die Anzahl der Stöße zwischen einem Alkaliatom und einem Stoßpartner wie $^4$He ist umso höher, je höher der Druck ist. Für $^4$He zum Beispiel ist die Verbreiterung der optischen Linienbreite des Alkaliatoms proportional zum Druck des Heliumgases. Außerdem besitzt $^4$He die wertvolle Eigenschaft, dass es nur wenig zerstörenden Einfluss auf die Polarisation der Alkaliatome hat. Bei der Polarisation von $^{129}$Xe wird daher regelmäßig mit einem Gasgemisch gearbeitet, welches zu 94 Prozent aus $^4$He besteht und einen Druck von etwa 1 MPa (10 bar) besitzt.

Bei dem gemäß Stand der Technik bekannten 100 Watt starken Laser für die Hyperpolarisation der Rb-Elektronen handelt es sich um einen glasfasergekoppelten Diodenlaser mit einer typischen Spektralbreite von 2 bis 4 Nanometern. Bei einem Gasdruck von 1 MPa (10 bar) ist die Linienbreite des optischen Überganges von Rubidiumatomen auf ca. 0,3 Nanometer verbreitert. Daher wird in den vorhandenen Rubidium-Xenon-Polarisatoren, in denen zum optischen Pumpen Hochleistungs-Diodenlaser mit typischerweise 2 Nanometer Linienbreite eingesetzt werden, nur ein Bruchteil des Laserlichts genutzt.

Die Partialdrucke von $^4$He betragen in dem Gasgemisch bis zu 1 MPa (10 bar). Im Vergleich zu den übrigen Partialdrucken (Xenon bzw. Stickstoff) ist dies sehr hoch. Dies soll bewirken, dass polarisierte Alkalimetall- oder Edelgasatome selten an die Innenwand der Glaszelle gelangen und dort z.B. durch Wechselwirkung mit paramagnetischen Zentren ihre Polarisation verlieren. Mit zunehmendem Partialdruck des $^4$He nimmt also die Wahrscheinlichkeit ab, dass polarisierte Atome nachteilhaft an die Zellinnenwand stoßen.

Ein polarisiertes Alkaliatom wie z.B. Rubidium vermag eine Fluoreszenzstrahlung zu erzeugen. Wird eine solche Strahlung von einem weiteren polarisierten Alkaliatom eingefangen, so führt dieser Einfang zur Depolarisation des Alkaliatoms. Der bei der Polarisation von Edelgasen eingesetzte Stickstoff im Gasgemisch dient dem Strahlungseinfang dieser Fluoreszenzstrahlung, um die vorgenannte unerwünschte Depolarisation herabzusetzen. Das Element Stickstoff im Gasgemisch weist ebenso wie Xenon nur einen geringen Partialdruck auf. Dieser Partialdruck beträgt typischerweise ca. 10 kPa (0,1 bar).

Die schweren Edelgas-Atome, wie z. B. Xenon-Atome, verursachen bei Stößen mit den Alkaliatomen eine starke Relaxation der Polarisation der Alkaliatome. Um die Polarisation der Alkaliatome beim optischen Pumpen so groß wie möglich zu halten, muss der Partialdruck des Xenongases im Gasgemisch entsprechend klein sein. Selbst bei einem Xenon-Partialdruck im Gasgemisch von 10 kPa (0,1 bar) braucht man Laserleistungen um die 100 Watt, um im ganzen Probenvolumen eine Polarisation der Alkaliatome von etwa 70 Prozent zu erreichen.

Beim Stand der Technik werden Probenzellen aus Glas eingesetzt, die aus einem Stück geblasen sind. Dies hat zur Folge, dass die Fenster, durch die das Licht des Lasers ein- und austritt, stets gekrümmt bzw. abgerundet sind. Es treten beim Eintritt oder Austritt des Lichts des Lasers unerwünschte, nachteilhafte Linseneffekte auf. Das Licht des Lasers wird fokussiert oder aufgeweitet. Hierdurch verschlechtert sich die der Polarisationsgrad erheblich. Der Querschnitt der Probenzelle wird also nicht gleichmäßig durch das Licht des Lasers ausgeleuchtet.

Ein Gasvolumen mit geeigneter Zusammensetzung wird gemäß Stand der Technik durch eine zylinderförmige Probenzelle gedrückt. Das Licht des Lasers, der die Polarisation erzeugt, wird in der Probenzelle absorbiert. Dabei durchstrahlt der Pumpstrahl die Probenzelle in Strömungsrichtung des Gemisches, welches die optisch pumpbare Spezies und die zu hyperpolarisierenden Atomkerne umfasst parallel zum Magnetfeld.

Aus US 2002/0107439 A1 ist bekannt, dass Laserlicht im Gegenstrom zu dem fließenden Gemisch in die Probenzelle einzustrahlen.

Nachteilig wird mit allen, bisher aus dem Stand der Technik bekannten Verfahren und Vorrichtungen zur Hyperpolarisation nur ein vergleichsweise geringer Polarisationsgrad der Kernspins von maximal etwa 40 % erzielt. Grund hierfür sind Wechselwirkungen in Form von Stößen des Alkalimetalls oder des Edelgases gegen die Innenwände der Probenzelle.

Aufgabe der Erfindung ist es daher, ein Verfahren zur Hyperpolarisation von Atomkernen und insbesondere Edelgaskernen bereit zu stellen, welches zur Erhöhung des Polarisationsgrades führt.

Es ist weiterhin Aufgabe der Erfindung eine Vorrichtung zur Durchführung des Verfahrens bereit zu stellen.

Die Aufgabe wird durch ein Verfahren mit der Gesamtheit der Merkmale des Anspruchs 1 und durch eine Vorrichtung gemäß Nebenanspruch gelöst. Vorteilhafte Ausgestaltungen ergeben sich aus den darauf rückbezogenen Ansprüchen.

Das erfindungsgemäße Verfahren sieht vor, eine mittels Laserlicht erzeugte Polarisation eines Elektronenspins einer optisch pumpbaren Spezies in einem Gemisch auf den Kernspin eines zu hyperpolarisierenden Atoms zu übertragen. Das Gemisch umfasst die optisch pumpbare Spezies, die zu hyperpolarisierenden Kerne und gegebenenfalls weitere Bestandteile, wie z.B. Puffer- und Quenschgase sowie gegebenenfalls eine weitere Alkalimetall-Sorte als Hilfs-Alkalimetall.

Die Bestandteile des Gemisches oder andere, für die Hyperpolarisation inerte Bestandteile, werden durch geeignete Ausgestaltung des Einlasses der Probenzelle für diese Bestandteile derartig in die Probenzelle geleitet, dass die optisch pumpbare Spezies und/oder die zu hyperpolarisierenden Kerne die Innenwände der Probenzelle nicht, oder in geringem Ausmaß berühren. Dadurch werden nachteilige Wechselwirkungen in Form von Stößen des Alkalimetalls und / oder des Edelgases gegen die Innenwände der Probenzelle vermieden, die ansonsten die Polarisation der Elektronen der optisch pumpbaren Spezies und der zu hyperpolarisierenden Kerne entlang des Querschnitts der Probenzelle verringern würden.

Es ist vorteilhaft möglich, für die Hyperpolarisation inerte Verbindungen, wie Puffergase derartig in die Probenzelle zu leiten, dass die Relaxation der optisch pumpbaren Spezies und die der hyperpolarisierten Atomkerne durch Stöße an der Innenwand vermieden wird.

Das Gemisch mit optisch pumpbarer Spezies und zu hyperpolarisierenden Kernen kann aber auch selbst als Freistrahl in die Probenzelle geleitet werden. Der Freistrahl umfasst das Gemisch. Deren Bestandteile berühren auf Grund der Ausführung in Form eines Freistrahls nicht die Innenwände der Probenzelle. Auch diese Maßnahme für sich allein bewirkt, dass die optisch pumpbare Spezies und/oder die zu hyperpolarisierenden Kerne die Innenwände der Probenzelle nicht berühren.

In Abhängigkeit vom Gasdruck und von der Fließgeschwindigkeit des Gemisches wird der Freistrahl und/oder der Mantelstrom so ausgestaltet und in die Probenzelle geleitet, dass die Verweilzeit der hyperpolarisierten optisch pumpbaren Spezies und die der hyperpolarisierten Kerne in der Probenzelle geringer ist, als deren Diffusionszeit zu den Innenwänden der Probenzelle.

Durch Ausbildung des Mantelstromes und/oder des Freistrahls wird also bewirkt, dass das Gemisch mit der hyperpolarisierten optisch pumpbaren Spezies und den hyperpolarisierten Kernen die Innenwände erst an der Stelle berühren kann, an der das Gemisch bereits wieder aus der Probenzelle zwecks Anreicherung geleitet wird.
Die Relaxation der optisch pumpbaren Spezies und/oder die der hyperpolarisierten Kerne durch Stöße an die Innenwände der Probenzelle wird auf diese Weise vollständig vermieden.

Der Freistrahl kann als dünne Schicht mit einer Dicke von z.B. 1 cm und/oder gegebenenfalls weniger als 1 cm Durchmesser ausgeformt werden.
Das Gemisch wird dann als ein freier Strahl in die Probenzelle injiziert und berührt dessen Innenwände nicht oder aber in weit geringerem Ausmaße als dies gemäß Stand der Technik durch einfaches Hindurchdrücken des Gemisches durch eine Probenzelle der Fall ist. Es treten keine Konvektionsströmungen an die Innenwände auf. Die Fließgeschwindigkeit eines Volumenelements beträgt z.B. etwa 0,5 cm je Sekunde und der Gasdruck in der Probenzelle z.B. 700-1500 kPa (7- 15 bar).

Hierzu weist die erfindungsgemäße Vorrichtung Mittel auf, die das Gemisch in die Probenzelle als Freistrahl injizieren, so dass das Gemisch nicht gegen die Wände stößt und also die Wandrelaxation vermieden wird. Ein derartiger Flusspolarisator wird im weiteren auch als Jet-Polarisator bezeichnet. Eine Düse ist als Mittel zur Ausbildung des Freistrahls vorgesehen. Die Düse steht mit der Gasversorgung der Probenzelle in Verbindung und weist Abmessungen auf, die einen Freistrahl des Gemisches erzeugen kann. Hierzu wird das Gasgemisch mit geeignetem Druck über die Düse in die Probenzelle injiziert.

Eine besonders einfache und wirkungsvolle optische Pumpzelle mit, im Vergleich zum Stand der Technik, verminderter Wandrelaxation sieht vor, den oder die Ein- und/oder Auslassstutzen anstelle eines 90° Winkels (zur Längsachse der Zelle) um einen definierten Winkel, insbesondere um 45° zu neigen.

Eine Gasversorgung umfasst die Leitungen und die Vorratsbehälter für eine bestimmte chemische Spezies, wie z.B. die optisch pumpbare Spezies und andere inerte Bestandteile, wie auch für die zu hyperpolarisierenden Kerne. Mehrere Gasversorgungen stellen die verschiedenen Gase bereit, die für die Hyperpolarisation einer bestimmten Sorte Kerne oder Edelgase benötigt werden. Es können Mischkammern zur Mischung der verschiedenen Bestandteile vorgesehen sein.

Die in die Probenzelle durch die Düse injizierten Bestandteile des Freistrahls berühren die Wände nicht, so dass die Wandrelaxation der optisch pumpbaren Spezies und der hyperpolariserten Kerne vermieden wird. Im Effekt bewirkt die Ausformung des Freistrahls durch die erfindungsgemäße Vorrichtung eine Anhebung des Polarisationsgrades während der Durchführung des Verfahrens.

Das Verfahren wird ferner so ausgeführt, dass der Abstand zwischen dem Ende des Freistrahls, an dem das Gemisch aus der Probenzelle geleitet wird, und dem oder den Eintrittsfenstern für das Laserlicht genügend groß gewählt wird, damit sich die Bestandteile des Gemisches, insbesondere die optisch pumpbare Spezies nicht an den Innenwänden des oder der Eintrittsfenster für das Laserlicht abscheiden können.

Auch diese Maßnahme für sich allein bewirkt eine deutliche Anhebung des Polarisationsgrades der hyperpolarisierten Atomkerne.

Das Eintrittsfenster der Probenzelle für das Laserlicht kann hierzu einen größtmöglichen Abstand zum Eingang der Probenzelle für die optisch pumpbare Spezies aufweisen.

Dadurch kann die Dichte der optisch pumpbaren Spezies sehr hoch gewählt werden, das heißt z.B. mindestens $10^{14}$ cm$^{-3}$ $^{85}$Rb-Atome und größer, ohne dass es zu den genannten nachteiligen Effekten der Abscheidung der optisch pumpbaren Spezies an dem oder den Eintrittsfenstern für das Laserlicht kommt.

Es wurde im Rahmen der Erfindung erkannt, dass sich bei Polarisatoren gemäß Stand der Technik, mit Ein- und Auslass für das Gemisch im rechten Winkel zu einer Längsseite der zylinderförmigen Probenzelle, auf der Innenseite des Eintrittsfensters für das Laserlicht auf Grund der räumlichen Nähe zum Eingang für das Gemisch mit der Zeit eine dünne Schicht der optisch pumpbaren Spezies ablagert, die beim Durchgang eines Pumpstrahls mit hoher Leistungsdichte verdampft, und eine Grenzschicht (Dicke < 0,3 mm) mit hoher Dichte ausbildet. Da die Atome in dieser Grenzschicht nahezu unpolarisiert sind, wird in dieser Grenzschicht ein großer Teil (> 50 %) der Leistung des Pumpstrahls absorbiert. Diese in der Grenzschicht absorbierte Leistung, steht für den eigentlichen Pumpprozess in der Zelle nicht mehr zur Verfügung. Darüber hinaus regt der auftretende starke Temperaturgradient gemäß Stand der Technik zwischen dieser Grenzschicht und dem übrigen Zellvolumen konvektive Strömungen in der Zelle an. Dadurch wird der Transport des Gases an die Zellwände beschleunigt und die Wandrelaxation nochmals vergrößert. Außerdem wird die Wandrelaxation der Kernspins durch den Kontakt mit der Grenzschicht dramatisch erhöht. Die Führung des Gemisches gemäß Stand der Technik erfolgt also U-förmig.

Das Verfahren kann erfindungsgemäß auch so ausgeführt werden, dass der Mantelstrom aus einer inerten Verbindung zur Separierung des Gemisches von den Innenwänden in die Probenzelle geleitet wird.

Das oder die Eintrittsfenster für den oder die Laser werden mit einem Mantelstrom gespült. Der Mantelstrom wird so in die Probenzelle geleitet, dass er den Freistrahl umgibt und die Innenwände der Probenzelle spült. Der Mantelstrom hat bezüglich der Hyperpolarisation keine nachteiligen Effekte. Vorteilhaft umfasst der Mantelstrom eine inerte Verbindung, die für die Hyperpolarisation eines bestimmten Kerns notwendig ist. Beispielhaft sei für die Hyperpolarisation von $^{129}$Xe ein Mantelstrom aus $^{4}$He genannt.

Der Mantelstrom wird durch Mittel erzeugt, die eine Düse und mindestens eine mit dieser in Verbindung stehende gesonderte Gasversorgungen umfassen, mit denen ein dünner Mantelstrom zur Spülung der Innenwände der Probenzelle erzeugt und in diese eingeleitet wird. Dadurch wird bewirkt, dass das Gemisch im Freistrahl nicht gegen die Wände stößt. Die Wandrelaxation der optisch pumpbaren Spezies und der Kerne wird dadurch verhindert.

Der Laser kann insbesondere im Falle einer zylinderförmigen Probenzelle so angeordnet sein, dass das Laserlicht im Gegenstrom, das heißt antiparallel zur Strömungsrichtung des in der Probenzelle fließenden Gemisches und des Magnetfeldes in die Probenzelle einstrahlt. Hierzu ist der Laser und die Polarisationsoptik an dem, dem Eingang des Gemisches mit der optisch pumpbaren Spezies gegenüberliegenden Ende der Probenzelle angeordnet.

Dann nimmt vorteilhaft die Pumplichtleistung und die Intensität des Lasers in Strömungsrichtung des Gemisches zu. Die Auskopplung des Gemisches erfolgt in diesem Fall vorteilhaft an der Stelle der Probenzelle, an der die Intensität des Lasers am größten ist.

Das Laserlicht kann in einer besonders vorteilhaften Ausgestaltung der Erfindung auch senkrecht zur Strömungsrichtung des in der Probenzelle fließenden Gemisches in die Probenzelle eingestrahlt werden. Dadurch wird besonders vorteilhaft bewirkt, dass der Freistrahl in seiner ganzen Ausdehnung gleichmäßig vom Laserlicht durchstrahlt wird. Der Vorteil besteht darin, dass der Pumplaser geometrisch gesehen eine geringe Transmissionslänge auf Grund der Dicke des Freistrahls durchdringen muss und somit eine gleichmäßig hohe Alkalimetallpolarisation entlang des Freistrahls erzielt wird.

Für derartige Verfahren kann die Vorrichtung mit mindestens einem Laser, vorzugsweise mit zwei Lasern und geeigneter Optik ausgeführt sein, die senkrecht zur Flussrichtung des Gemisches im Freistrahl angeordnet sind.

Durch entsprechende Anordnung der Laser und der jeweils zugehörigen Polarisationsoptik in der Vorrichtung wird vorteilhaft bewirkt, dass auch mit zunehmender Kernspinpolarisation am Ausgang der Probenzelle die Polarisation der Elektronen effektiv auf die zu hyperpolarisierenden Kerne übertragen wird.

Durch diese Maßnahme für sich allein, wird unabhängig von der Ausbildung eines Freistrahls und/oder eines Mantelstroms, eine weitere Erhöhung des Polarisationsgrades der Kernspins des aus der Zelle austretenden Gemisches gegenüber einem Flusspolarisator mit Einstrahlrichtung des Laserlichts in oder entgegen der Flussrichtung des Gemisches erzielt.

In einer weiteren besonders vorteilhaften Ausgestaltung der Erfindung werden während des Verfahrens die Wände der Probenzelle gekühlt. Hierzu kann die Vorrichtung z.B. eine Thermobox umfassen. Eine Vorrichtung umfassend Mittel zum Kühlen der Wände, steht im Gegensatz zu den bisherigen Vorrichtungen, wie Flusspolarisatoren, bei denen die Wände immer mitgeheizt werden. Ermöglicht wird dies durch gesonderte Heizungen für die Bestandteile des Gemisches vor dem Eingang der Probenzelle und dem gebildeten Freistrahl, da mit diesem die Wandberührung des optisch gepumpten Gases während der Durchgangszeit verhindert wird.

Vorteilhaft wird durch diese Maßnahme bewirkt, dass die Wärmeabfuhr aus dem Gasgemisch optimiert wird. Besonders vorteilhaft wird auch bewirkt, dass keine unpolarisierten Atome wie Alkalimetalle mehr von den Innenwänden abgegeben werden.

In einer weiteren Ausgestaltung der Erfindung wird der Spinaustausch während des Verfahrens indirekt auf den Kernspin eines zu hyperpolarisierenden Kerns übertragen. Der Spinaustausch wird dabei zunächst von den Elektronen einer optisch pumpbaren Spezies auf die Elektronen mindestens einer nicht durch den oder die Laser optisch pumpbaren Spezies übertragen und von dort auf die Kerne der zu hyperpolarisierenden Kerne übertragen. Das Laserlicht pumpt die nicht optisch pumpbare Spezies nicht. Dann wird vorteilhaft eine höhere Dichte der nicht optisch pumpbaren Spezies gegenüber der optisch pumpbare Spezies gewählt. Weiterhin wird vorteilhaft bewirkt, dass eine nicht optisch pumpbare Spezies zur Übertragung der Elektronenspinpolaristion auf den Kernspin gewählt werden kann, die eine hohe Effizienz, insbesondere eine Effizienz nahe 1 hierfür aufweist.

Die Vorrichtung zur Hyperpolarisation umfasst zu diesem Zweck mindestens zwei Vorratsbehälter für die optisch pumpbare Spezies und die nicht optisch pumpbare Spezies, wodurch diese auch für indirektes Spinaustausch optisches Pumpen genutzt werden kann. Die Vorratsbehälter sind vorteilhaft jeweils in einer separaten Gasversorgung der Vorrichtung angeordnet und mit eigenen Heizungen versehen.

Die Vorrichtung ist weiterhin so ausgeführt, dass das Eintrittsfenster der Probenzelle für das Laserlicht einen größtmöglichen Abstand zum Eingang der Probenzelle für die optisch pumpbare Spezies aufweist.

Dadurch wird vorteilhaft bewirkt, dass die optisch pumpbare Spezies sich nicht am Eintrittsfenster abscheidet.

Das Eintrittsfenster der Probenzelle für das Laserlicht kann besonders vorteilhaft einen so großen Abstand zum Ausgang der Probenzelle für das Gemisch aufweisen, dass eine Abscheidung der optisch pumpbaren Spezies an der Innenwand des Eintrittsfensters für das Laserlicht vermieden wird.

Über Absaugrohre und Leitungen wird das Gemisch radial zur Probenzelle abgesaugt. Es handelt sich demgemäß um einen Durchfluss-Polarisator.

Die Ausbildung eines Mantelstroms, des Freistrahls, die genannten Mindestabstände des Gemisches von den Innenwänden in Abhängigkeit von der Fließgeschwindigkeit und des Drucks, sowie geeignete Laseranordnungen bewirken je für sich allein schon, dass Wechselwirkungen des Gemisches mit den Innenwänden Probenzelle vermieden werden und eine Anhebung des Polarisationsgrades während des Verfahrens erzielt wird. In Kombination miteinander kann eine weitere Erhöhung des Polarisationsgrades der zu hyperpolarisierenden Kerne erreicht werden.

Die Spulen zur Erzeugung eines Magnetfeldes sind so angeordnet, dass die Magnetfeldrichtung entweder entgegen oder in Richtung des Laserstrahls liegt.

Als optisch pumpbare und gegebenenfalls nicht optisch pumpbare Spezies werden insbesondere Alkalimetalle gewählt, da diese ein großes Dipolmoment aufweisen.

Das erfindungsgemäße Verfahren ist z.B. zur Hyperpolarisation von $^{129}$Xe mittels $^{85}$Rb allein, oder mittels $^{85}$Rb als optisch pumpbare und eines Cäsiumisotops als nicht optisch pumpbare Spezies besonders geeignet. Es kann mit $^{85}$Rb und / oder des Cäsiumisotops aber auch $^{13}CO_2$ hyperpolarisiert werden.

Das Gemisch wird entweder kontinuierlich oder halbkontinuierlich durchgeleitet durch den Durchfluss-Polarisator geleitet. Im Fall von $^3$He wird ein Polarisator mit abgeschlossener Probenzelle verwendet.

Im weiteren wird die Erfindung an Hand von Ausführungsbeispielen und der beigefügten Figuren näher beschrieben.

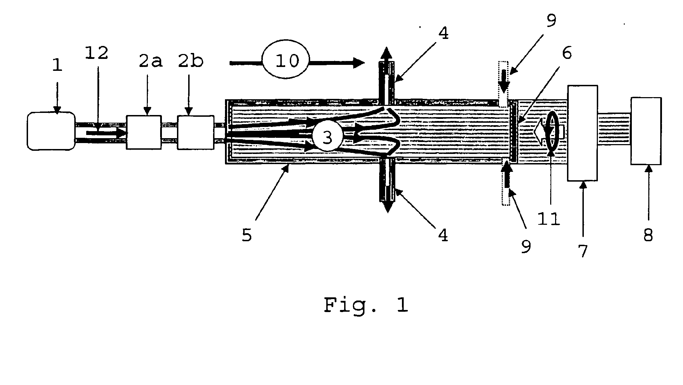

In Fig. 1 ist eine erfindungsgemäße Vorrichtung zur Ausbildung des Freistrahls dargestellt, die einfach und kompakt aufgebaut ist.

Der Injektions(Jet-)polarisator in Fig. 1 ist mit einer einzigen Gasversorgung 1 versehen. Die Gasversorgung 1 gewährleistet die Zuführung aller für die Hyperpolarisation notwendigen Gase. Ausgehend von der Gasversorgung 1 durchströmt das Gasgemisch 12, dargestellt durch den fettgedruckten Pfeil, nacheinander zwei Vorratsbehälter 2a, b für zwei verschiedene Alkalisorten. Im ersten Vorratsbehälter 2a ist ein Alkalimetall als optisch pumpbare Spezies enthalten, im zweiten Vorratsbehälter 2b ist eine nicht optisch pumpbare Spezies enthalten oder umgekehrt. Ein Laser 8 mit Polarisationsoptik 7 ist so angeordnet, dass das Laserlicht 11 entgegen der Strömungsrichtung des Freistrahls 3 durch das Eintrittsfenster 6 in die Probenzelle 5 eintritt.

Die Vorrichtung ist geeignet für indirektes Spinaustausch optisches Pumpen z.B. mit einem optisch pumpbaren Alkalimetall und einem nicht optisch pumpbaren Alkalimetall.

Gemäß Fig. 1 wird das Gasgemisch 12 mit z.B. $N_2$, $^4$He und $^{129}$Xe in den Vorratsbehältern 2a, b mit Alkalidämpfen angereichert und expandiert über eine kreisförmige Düse mit einem Durchmesser von 1 cm (nicht dargestellt) in Form eines Freistrahls 3 in die Probenzelle 5. Der Freistrahl ist so dimensioniert, dass die Bestandteile des Gasgemisches bei vorgegebenem Druck des Gasgemisches von etwa 500 bis 1000 kPa (5 bis 10 bar) während der Durchlaufzeit des Freistrahls die Innenwände der Probenzelle 5 nicht berühren. Hierdurch wird die Wandrelaxation der optisch pumpbaren Spezies und der hyperpolarisierten Atomkerne vermieden.

Das Gasgemisch 12 im Freistrahl 3 wird über insgesamt sechs Absaugrohre 4 radial abgesaugt, so dass es vorteilhaft nicht bis zum Eintrittsfenster 6 für das Laserlicht 11 gelangt und dort auch nicht zur Abscheidung der optisch pumpbaren Spezies kommt. Die hyperpolarisierten Kerne werden sodann angereichert.

Der optische Pumpstrahl 11 wird hier im Gegenstrom, das heißt antiparallel entgegen der Strömungsrichtung des Freistrahls 3 und longitudinal zu einem Magnetfeld 10 in die Probenzelle 5 eingestrahlt.

Optional kann zur Vermeidung der Belegung des Eintrittsfensters 6 für den Pumpstrahl mit Alkalimetallen das Eintrittsfenster 6 mit einem Mantelstrom 9 aus einem inertem Gas, z.B. $^4$He gespült werden. Hierzu sind nahe des Eintrittsfensters 6 zusätzliche Einlasse für den Mantelstrom 9 angeordnet.

Die Bauweise gemäß Fig. 1 ist besonders kompakt.

Der Laser 8 und die Polarisationsoptik 7 kann aber auch so angeordnet sein, dass der Pumpstrahl den Freistrahl 3 senkrecht zur Strömungsrichtung des Freistrahls 3 durchstrahlt. Diese Bauweise ist zwar nicht so kompakt wie die gemäß der Fig. 1. Dafür ist vorteilhaft gewährleistet, dass der Freistrahl 3 über sein gesamtes Volumen eine homogene Alkalimetall-Polarisation aufweist, insbesondere wenn der Freistrahl 3 mit nur wenigen Millimetern Höhe und / oder Dicke dimensioniert ist.

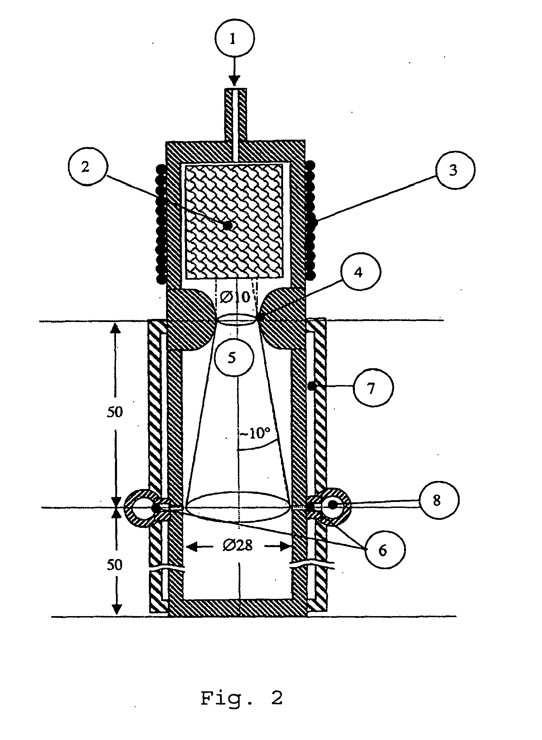

Der Freistrahl kann genauso gut mit nur einer optisch pumpbaren Spezies und der zu hyperpolarisierenden Kerne ausgebildet werden. In diesem Fall benötigte man eine Vorrichtung ohne einen Vorratsbehälter für eine nicht optisch pumpbare Spezies. In Fig. 2 ist eine derartige Vorrichtung zur Ausbildung des Freistrahls 5 gezeigt, die zu einem erhöhten Polarisationsgrad durch Vermeidung der Wandrelaxation führt, wobei nur eine optisch pumpbare Spezies neben den anderen Bestandteilen in die Probenzelle geführt wird. Die Durchmesserangaben sind in Millimetern bemessen. Der Gasdruck in der Zelle beträgt etwa 700 bis 1500 kPa (7 bis 15 bar).

Fig. 2 zeigt im übrigen:

1 Gaszufuhr
2 Mit Alkali-bedampfter poröser Metallkörper oder -geflecht (Cu, Pt, etc.)
3 Heizung (Mikrowellen, Licht, Heißluft)
4 Düse
5 Freistrahl
6 Absaugkanäle für den Freistrahl
7 Gas- oder Wasserkühlung
8 Sammelkanal für polarisiertes Gas

Fig. 3 zeigt schematisch eine Vorrichtung mit zwei Vorratsbehältern 34a und 34b für eine optisch pumpbare Spezies, z.B. $^{85}$Rb und für eine nicht optisch pumpbare Spezies, z.B. Cäsium. Auch diese Vorrichtung ist geeignet für indirektes Spinaustausch optisches Pumpen z.B. mit einem optisch pumpbaren Alkalimetall und einem nicht optisch pumpbaren Alkalimetall.

Ausgehend von den voneinander unabhängigen Gasversorgungen 31a und 31b wird ein Gasstrom z.B. mit $N_2$, $^4$He und $^{129}$Xe durch die Strömungsmesser 32a und 32b sowie den Nadelventilen 33a und 33b unabhängig voneinander gesteuert und den Vorratsbehältern 34a und 34b zugeführt.

In den Vorratsbehältern werden die Gase mit Alkalidampf angereichert. Jeder Vorratsbehälter weist eine eigene Heizung 35a und 35b auf. Dadurch wird abhängig von der Wahl der optisch pumpbaren Spezies in Vorratsbehälter 34a und der nicht optisch pumpbaren Spezies in Vorratsbehälter 34b die Volumengasströme kontrolliert in die Mischkammer 36 geleitet.

Durch die unabhängig voneinander eingerichteten Heizsysteme 35a und 35b wird vorteilhaft bewirkt, dass für die optisch pumpbare Spezies A1 und die nicht optisch pumpbare Spezies A2 unterschiedliche Dampfdruckdichten und Temperaturen eingestellt werden können. Dies führt zur weiteren Optimierung des Verfahrens.

In der Mischkammer 36 werden die Gase miteinander gemischt und von dort in die Pumpzelle 39 expandiert. Die optisch pumpbare Spezies A1 und die nicht optisch pumpbare Spezies A2 kommen folglich erst in der Mischkammer 36 miteinander in Kontakt. Von der Mischkammer 36 gelangt das Gasgemisch 37 in die Pumpzelle 39, in der die Atomkerne durch indirektes Spinaustausch optisches Pumpen hyperpolarisiert werden. Die Vorrichtung dient der besseren Kontrolle des Mischungsverhältnisses der Alkalisorten.

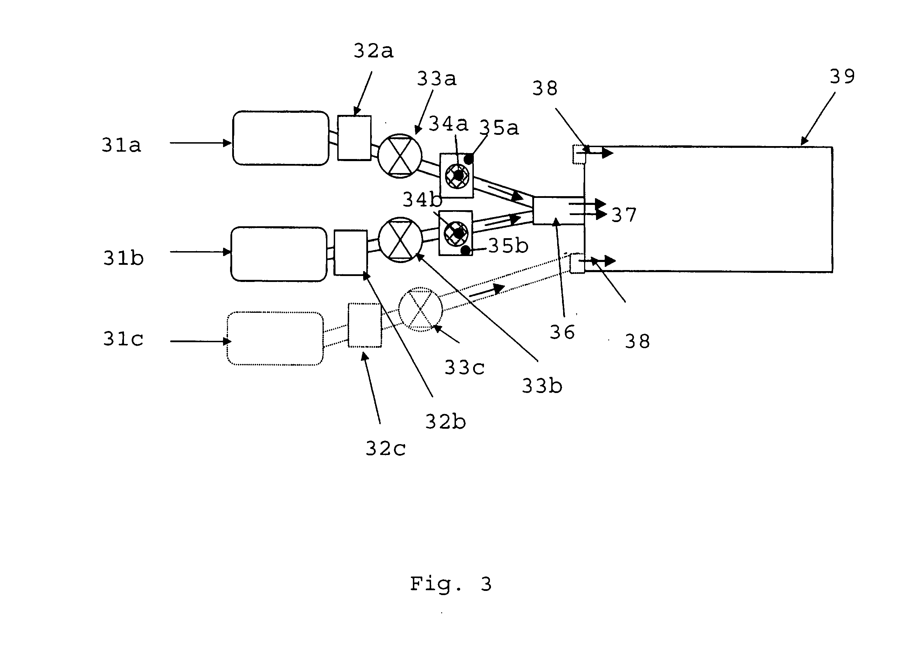

In Figur 3 ist die Erzeugung eines $^4$He-Mantelstroms 38 dargestellt, mit der allein der erfindungsgemäße Effekt erzielt wird, die Hyperpolarisation ohne Wandberührung der optisch pumpbaren Spezies und/oder der hyperpolarisierten Atomkerne durchzuführen. Der Mantelstrom 38 gewährleistet eine verbesserte Wärmeabfuhr und eine alkalifreie Atmosphäre an den Innenwänden der Probenzelle 39. Allerdings kann das Gasgemisch 37 selbstverständlich zusätzlich auch als Freistrahl ausgeführt werden. Zur Erzeugung des Mantelstromes 38 ist eine eigene Gasversorgung 31c sowie ein Strömungsmesser 32c und ein Nadelventil 33c vorgesehen. Zwei Einlässe am oberen und unteren Teil der Probenzelle 39 sind dargestellt, von denen aus Platzgründen nur der untere mit der genannten Gasversorgung in Kontakt stehend eingezeichnet wurde. Selbstverständlich steht auch der obere Einlass 38 mit einer gegebenenfalls separaten Gasversorgung in Verbindung. In dem Fall, dass der Laser im Gegenstromprinzip angeordnet ist, kann der Mantelstrom so in die Probenzelle geführt werden, dass das Eintrittsfenster für den Laser mit dem Mantelstromes gespült wird. In der Fig. 3 wurde die Laseranordnung allerdings nicht dargestellt, ebenso wenig der Ausgang des Polarisators für das Gasgemisch.

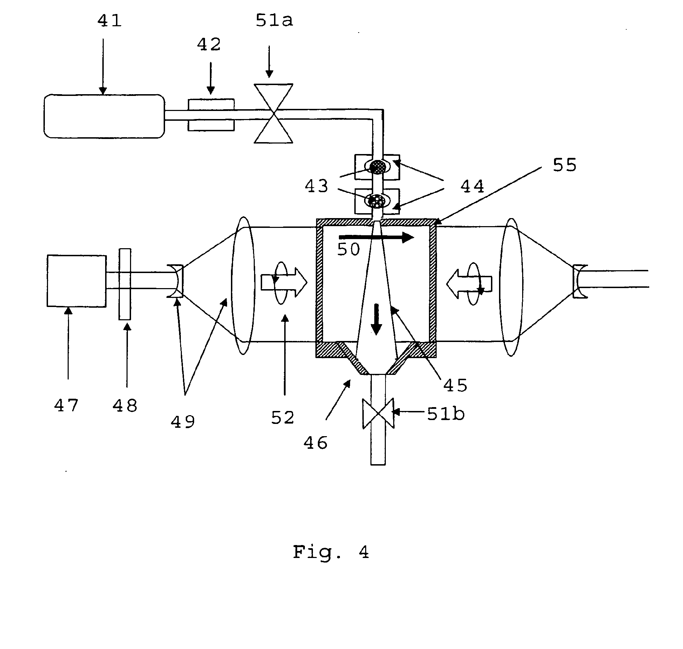

In Figur 4 ist schematisch eine Anordnung dargestellt, mit der zwei Sorten Alkalispezies A1 und A2 kontrolliert in eine Probenzelle 55 geführt werden, wobei die Wandrelaxation durch den ausgeführten Freistrahl 45 vollständig verhindert wird.

Die Vorrichtung ist wie die der Fig. 1 für indirektes Spinaustausch optisches Pumpen oder auch für einfaches indirektes Spinaustausch optisches Pumpen geeignet. In letztgenanntem Falle, wird auf je einen Behälter 43 und eine Heizung 44 für die nicht optisch pumpbare Spezies verzichtet.

Ein Gasgemisch aus z.B. $^{129}$Xe, $^4$He und $N_2$ wird aus einer Gasversorgung 41 über den Strömungsmesser 42 und das Nadelventil 51a in die beiden Vorratsbehälter 43 geleitet und dort mit den Alkalidämpfen A1 und A2 angereichert. Das Verhältnis der Konzentrationen der beiden Alkalispezies wird durch getrennte Heizungen 44, z.B. HF-Heizungen der Vorratsbehälter 43 kontrolliert. Als optisch pumpbare Spezies kann $^{85}$Rb und als nicht optisch pumpbare Spezies ein Cäsiumisotop gewählt werden. Der Volumenstrom wird mit den Nadelventilen 51a, 51b am Ein- und Ausgang der Probenzelle 55 gesteuert. Zu diesem Zweck ist vor den Vorratsbehältern 43, 44 der Strömungsmesser 42 angeordnet. Es können für jeden Vorratsbehälter 43, 44 auch eigene Gasversorgungen und Ventile vorgesehen sein.

Das mit Alkaliatomen angereicherte Gasgemisch strömt durch eine Düse am Eingang der Probenzelle 55 in diese und bildet dort auf Grund der Abmessungen der Düse, die wie in der Fig. 2 ausgeführt sein kann, einen Freistrahl 45 aus.

Der laminare Freistrahl 45 wird von den zwei senkrecht zur Strömungsrichtung des Freistrahls 45 angeordneten Lasern vollständig und daher mit extrem hoher Pumpleistung durchstrahlt. Nur der linke Laser 47 ist zusammen mit seiner Polarisationsoptik 48 und dem Teleskop mit Zylinderlinsen 49 in der Figur aus Platzgründen wiedergegeben, wohingegen von dem rechten Laser nur das Teleskop mit der Zylinderlinse ohne Bezugszeichen wiedergegeben ist. Das zirkular polarisierte Laserlicht 52 tritt durch die beiden vertikalen Eintrittsfenster in die Probenzelle 55 ein und durchstrahlt den Freistrahl 45 vollständig.

Der Freistrahl 45 wird als dünne Schicht mit einer Höhe von ca. 20 mm und einer Dicke von ca. 1 mm bei Eintritt in die Probenzelle 55, und einer Höhe von ca. 32 mm und einer Dicke von ca. 13 Millimeter am Austritt 46 aus der Probenzelle 55 ausgeformt. Dadurch wird vorteilhaft bewirkt, dass die Dichte der optisch gepumpten Alkalispezies hoch sein kann, da die beiden senkrecht angeordneten Laser maximal nur eine Schicht von ca. 1 cm Durchmesser durchdringen müssen. Ein weiterer Vorteil ist, dass wegen der geringen Schichtdicke des Freistrahls 45, die Absorption von Fluoreszenzstrahlung vermindert ist. Darüber hinaus ergibt sich gegenüber einem zylindersymmetrischen Strahl des Gemisches eine erhöhte Relaxation der zum Quenchen der angeregten Alikaliatome benutzten $N_2$-Moleküle durch eine erhöhte Wärmeabfuhr durch die große Grenzfläche zwischen Freistrahl 45 und dem Gas der Umgebung.

Die Gefahr, dass die vertikalen Eintrittsfenster für den Laserstrahl mit Alkalimetallen belegt werden, ist gering, wenn genügend Abstand zwischen dem Freistrahl 45 und den vertikalen Eintrittsfenstern für das Laserlicht gehalten wird. Die Strömung des Freistrahls 45 muss zum Ausströmen aus der Probenzelle 55 vorteilhaft nicht mehr umgelenkt werden. Ein weiterer Vorteil des Spinaustausch-Optischen Pumpens mit einer Freistrahlanordnung besteht darin, dass es nicht notwendig ist, die gesamte Probenzelle 55 zu beheizen, wie in Fig. 2 dargestellt. Es wird nur die Wärmemenge zugeführt, die zur Verdampfung des oder der Alkalimetalle und zur Aufheizung des Gasstroms erforderlich ist. Daher muss auch nur diese Wärme an die Umgebung abgeführt werden. Dieses ermöglicht eine wesentlich kompaktere Bauweise gegenüber den bisher bekannten Anordnungen.

Ein Magnetfeld 50 ist durch Anordnung einer oder mehrerer Spulen ebenfalls senkrecht zum Freistrahl 45 ausgerichtet.

Der Freistrahl 45 kann durch zwei nicht dargestellte, senkrecht zum Freistrahl 45 angeordnete Schilder nahe des Ausgangs 46 zur Vermeidung von Rückströmen zur Austrittseite des Polarisators hindurchgeleitet werden.

Der Freistrahl 45 kann zwecks Anreicherung hinter dem Ventil 51 b auf der Austrittseite der Zelle 55 in ein kaltes Lösungsmittel geleitet werden, das eine große Löslichkeit für das in der Zelle 55 hyperpolarisierte Gas besitzt.

Die Moleküle im Freistrahl 45 haben während des gesamten Polarisationsprozesses in der Probenzelle 55 keinerlei Wandkontakte.

In der Figur 4 ist eine Anordnung darstellt, bei der die optische Achse der Pumpstrahle 52 und das Magnetfeld 50 senkrecht zur Strömungsrichtung des Freistrahls 45 steht. Dieses stellt aus den beschrieben Gründen die optimale Lösung für einwandfreies Spinaustausch optisches Pumpen dar. Es sind jedoch auch die in den vorherigen Figuren gezeigten Konfigurationen möglich, die die wesentlichen Vorteile des berührungslosen Pumpens beibehalten, dabei aber einfacher und kompakter realisierbar sind.

Die gezeigten Vorrichtungen der Fig. 1 bis 4 und das Freistrahlverfahren können ohne Einschränkung der Erfindung auch für einfaches optisches Pumpen ausgebildet sein, das heißt ohne indirektes Spinaustausch-Pumpen verwendet werden. In diesem Fall benötigt man nur Vorrichtungen mit nur einem einzigen Vorratsbehälter für eine optisch pumpbare Spezies. Alle Vorrichtungen können mit Thermoboxen für die Probenzelle versehen sein. Es ist ohne jegliche Einschränkung der Erfindung möglich, die genannten Vorrichtungen mit Mitteln zur Ausgestaltung eines Mantelstromes zu versehen.

Das oben genannte indirekte Spinaustausch optische Pumpen lässt sich für beliebige Systeme wie folgt beschreiben:

$$P_{A1}(z) = s_z \cdot \frac{R(z)}{R(z) + \gamma_{sd}^{A1}} \qquad (1)$$

$$P_{A2}(z) = P_{A1}(z) \cdot \frac{\gamma_{ex}^{A2,A1}}{\gamma_{ex}^{A2-A1} + \gamma_{sd}^{A2}} \qquad (2)$$

$$\gamma_{ex}^{A2,A1} \cong \left\langle \sigma_{A2,A1} \cdot \mathbf{v} \right\rangle \cdot [A1] \qquad (3)$$

mit

$P_{A1}(z)$ volumengemittelter Polarisationsgrad der Elektronen der optisch pumpbaren (Alkali)Spezies A1,

$\gamma_{sd}^{A1}$ Elektronenspinrelaxationsrate für die optisch pumpbare Spezies A1,

$P_{A2}(z)$ volumengemittelter Polarisationsgrad der Elektronen der nicht optisch pumpbaren (Alkali)Spezies A2,

$s_z$ Photonenspin

$R(z)$ Optische Pumprate pro Alkalimetallatom an der Stelle z

$\gamma_{ex}^{A2,A1}$ Spinaustauschrate optisch pumpbare Spezies A1 auf nicht optisch pumpbare Spezies A2, und

$\gamma_{sd}^{A2}$ Elektronenspinrelaxationsrate für die nicht optisch pumpbare Spezies A2.

Insbesondere wenn [A2] » [A1] gilt weiterhin:

$$P_{EG}(z) \sim P_{A2}(z) \cdot \frac{\gamma_{se}^{EG,A2}}{\gamma_{se}^{EG,A2} + \gamma_W} \qquad (4)$$

und
mit

$$\gamma_{se}^{A2} = \left\langle \sigma_{EG,A2} \cdot \mathbf{v} \right\rangle \cdot [A2] \qquad (5)$$

$P_{EG}$ volumengemittelter Polarisationsgrad der zu hyperpolarisierenden Kerne (Edelgas),

$\gamma_{se}^{EG,A2}$ Spinaustauschrate von nicht optisch pumpbarer Spezies Spezies A2 auf den Kernspin EG,

$\gamma_W$ Wandrelaxationsrate der Kernspins,

$\sigma_{EG,A2}$ Wirkungsquerschnitt für die nicht optisch pumpbare Spezies A2 und den zu hyperpolarisierenden Kern EG,

$\sigma_{EG,A2}*v$ Mittelwert des Wirkungsquerschnitts über die Relativgeschwindigkeit v.

Der Index "ex" für exchange bezieht sich auf den Spinaustausch zwischen optisch pumpbarer A1 Spezies und nicht optisch pumpbarer Spezies A2. Der Index "se" für spinexchange bezieht sich auf den Spinaustausch zwischen nicht optisch pumpbarer Spezies A2 und dem Atomkern Ed.

Die allgemein für indirektes Spinaustausch optisches Pumpen angegebenen Gleichungen zeigen, dass unter der Voraussetzung, dass die Elektronenspinrelaxationsrate der optisch pumpbaren Spezies $\gamma_{sd}^{A1}$ kleiner als die Pumprate R des Lasers gehalten wird, es zum Effekt der Hyperpolarisation des Atomkerns kommt, sofern die Elektronenspinrelaxationsraten $\gamma_{sd}^{A1}$ und $\gamma_{sd}^{A2}$ kleiner als die Spinaustauschrate $\gamma_{ex}^{A2,A1}$ zwischen optisch pumpbarer Spezies A1 und nicht optisch pumpbarer Spezies A2 sind.

Da der Spinaustausch zwischen der optisch pumpbaren Spezies A1 und der nicht optisch pumpbaren Spezies A2 und gegebenenfalls weiterer Spezies Spin erhaltend ist und einen großen Wirkungsquerschnitt besitzt, kann die Spinpolarisation der Alkalimetall-Spezies A1, die optisch gepumpt wird, effektiv auf die Atome der Spezies A2, die nicht optisch gepumpt wird, übertragen werden. Dieses gilt auch, wenn die Dichte der Alkalimetall-Spezies A2 höher ist als die Dichte der Spezies A1 ist.

Der Begriff Wirkungsquerschnitt ist dabei ein Maß für die Wahrscheinlichkeit der Wechselwirkung der unterschiedlichen Teilchen und des Eintretens der Kernspinpolarision des Edelgases. Er ist definiert als Quotient aus der Zahl der Prozesse, die während einer bestimmten Zeit erfolgen, durch die Zahl der Teilchen, die je Flächeneinheit während dieser Zeit in das Reaktionsgebiet einfallen.

Die Gleichungen gelten für beliebige Systeme. Das heißt, dass die Gleichungen sowohl die Hyperpolarisation von Edelgaskernen mit zwei Spezies von Alkalimetallen umschreiben, als auch die der Hyperpolarisation von $^6$Li, $^{13}$C und anderer Kerne mittels geeigneten optisch pumpbaren und nicht optisch pumpbaren Spezies.

Dadurch kann der Gasdurchsatz und damit auch die Produktionsmenge der zu hyperpolarisierenden Kerne erhöht werden.

Beispielhaft kann für das Rubidium-Cäsium-Xenon-System folgende Berechnung aufgestellt werden:

Die Dichte der Rubidium-Atome als optisch pumpbare Spezies A1 in der Pumpzelle beträgt $10^{14}$ cm$^{-3}$. Die Leistung des Lasers sei R = $10^6$ s$^{-1}$. Die Elektronenspinrelaxationsrate $\gamma_{sd}^{Rb}$ von Rubidium als optisch pumpbare Spezies A1 beträgt bei einem Partialdruck des $^{129}$Xe von, 10 kPa (0,1 bar) ca. $3*10^4$ s$^{-1}$.

Der Laser leuchtet die Pumpzelle optimal aus, da die Dichte von Rubidium gering genug ist. Das heißt, die Dichte der optisch gepumpten Alkalispezies A1 wird so gewählt, dass der optische Pumpstrahl mit genügender Tiefe in das Pumpmedium eindringt.

Der Elektronenspin der Rubidiumatome wird polarisiert, siehe Gleichung (1). Durch die vergleichsweise niedrige Dichte der Rubidiumatome ist der volumengemittelte Polarisationsgrad $<P_{Rb}>$ (z) maximal, das heißt es ergibt sich hierfür ein Wert von 90%, da die Photonenspinpolarisation $s_z$ einen Wert von etwa 97% aufweist.

Zur Steigerung des Polarisationsgrades des $^{129}$Xe ist somit ein möglichst maximaler Polarisationsgrad von Rubidium als optisch pumpbarer Spezies Voraussetzung, wobei die Pumprate des Lasers größer als die Elektronenspinrelaxtionsrate der optisch pumpbaren Spezies (hier: R(z) > $\gamma_{sd}^{Rb}$) gehalten wird. Ist, wie im angegebenen Beispiel, die Rubidium-Dichte $n_{Rb}$ in der Pumpzelle zu hoch, fällt der Polarisationsgrad von Rubidium mit dem Ort z rasch ab.

Bei Rubidiumdichten größer $10^{14}$ cm$^{-3}$ im Pumpmedium tritt der bereits beschriebene Effekt der erhöhten Wandrelaxation auf Grund unpolarisierten Rubidiums, welches sich an der Wand abschlägt, auf.

Die Dichte von Cäsium-Atomen als nicht optisch pumpbarer Spezies A2 beträgt dagegen $10^{15}$ cm$^{-3}$. Die Dichte der optisch pumpbaren Spezies A1 (Rubidium) ist also um den Faktor 10 geringer gewählt, als die der nicht optisch pumpbaren Spezies A2 (Cäsium). Die Elektronenspinrelaxationsrate $\left(\gamma_{sd}^{Cs}\right)$ von Cäsium als nicht optisch pumpbarer Spezies A2 liegt in der gleichen Größenordnung, wie die des Rubidium. Allerdings ist die Effizienz zur Hyperpolarisation von $^{129}$Xe für Cäsium grö-ßer als für Rubidium.

Zur Bestimmung des Polarisationsgrades der Elektronen der nicht optisch pumpbaren Spezies A2 (Gleichung 2), hier der Cäsium-Atome, wird bei einer Temperatur von 170 °C in der Probenzelle, und einer Relativgeschwindigkeit von Rubidium und Cäsium von jeweils $2*10^4$ cm*s$^{-1}$ der Wirkungsquerschnitt zu etwa $2*10^{-14}$ cm$^{-2}$ und als Mittelwert über die Relativgeschwindigkeit $<\sigma_{Rb-Cs}*v>$ zu $4*10^{-10}$ cm$^3$s$^{-1}$ bestimmt. Die Spinaustauschrate $\gamma_{ex}^{Cs,Rb}$ zwischen Rubidium und Cäsium wird durch Lösen von Gleichung (3) zu $5*10^5$ s$^{-1}$ bestimmt.

Die optische Pumprate R und die Spinaustauschrate zwischen A1 und A2 müssen größer sein, als die Elektronenspinrelaxationsraten von Rubidium und Cäsium. Die Austauschrate zwischen Cäsium und Xenon muss größer sein, als die Kernspinrelaxationsrate des Xenon ($\gamma_w$).

Der volumengemittelte Polarisationsgrad der nicht optisch pumpbaren Spezies $<P_{Cs}>$ (z) beträgt nach Einsetzen in Gleichung (2) somit 85%.

Da der Spinaustausch zwischen den Alkaliatomen Rubidium und Cäsium Spin erhaltend ist und einen großen Wirkungsquerschnitt besitzt, kann die Spinpolarisation von der optisch pumpbaren Spezies Rubidium effektiv auf die Atome der nicht optisch pumpbaren Spezies Cäsium übertragen werden, auch wenn diese in höherer Dichte vorliegt.

Analog der Gleichungen (2) und (3) kann gemäß Gleichungen (5) und (4) die Spinaustauschrate $\gamma_{ex}^{Xe,Cs}$ und der volumengemittelte Polarisationsgrad von $^{129}$Xe ermittelt werden.

Die nicht optisch pumpbare Spezies wird im vorliegenden Fall so gewählt, dass der Wirkungsquerschnitt $\sigma_{Xe,Cs}$ für die Spinübertragung auf den Kern der Edelgasatome größer ist, als der der optisch pumpbaren Spezies. Die Spinaustauschrate $\gamma_{ex}^{Cs,Xe}$ zwischen nicht optisch pumpbarer Spezies Cäsium und zu hyperpolarisierenden Kernen des $^{129}$Xe beträgt gemäß Gleichung (5) somit 0,3 s$^{-1}$. Dieser Wert ist viel größer als die Verluste durch Wandrelaxation $\gamma_w^{Xe}$. Danach benötigt man zur Hyperpolarisation eines Xenon-Atoms ca. 3 Sekunden im Vergleich zu 50 Sekunden nach dem Stand der Technik mittels einfachen optischen Pumpens. Danach können Freistrahl und Fließgeschwindigkeiten in der Probenzelle abgestimmt sein.

Es können ohne Einschränkung der Erfindung auch andere Gasgemische in einer Probenzelle hyperpolarisiert werden. Beispielhaft seien die nachfolgenden Gemische genannt. Das erste Alkalimetall stellt jeweils die optisch pumpbare Spezies, das zweite die nicht optisch pumpbare Spezies im Gemisch dar:

- ein $^{85}$Rb-Li(Na, K)-Gemisch zur Hyperpolarisation von $^3$He.

- ein K-Li-Gemisch zur Hyperpolarisation von $^3$He.

Diese beiden Gemische werden in einer abgeschlossenen Probenzelle mittels indirektes Spinaustausch optisches Pumpen ohne Freistrahl oder Mantelstrom hyperpolarisiert.

Weiterhin kann mit dem erfindungsgemäßen Verfahren ein $^{85}$Rb-Cs-Gemisch neben der Hyperpolarisation von $^{129}$Xe auch für die Hyperpolarisation von $^{13}CO_2$ verwendet werden. Für die einfache Hyperpolarisation von $^{13}CO_2$ sind Rb- oder Cs-Isotope grundsätzlich geeignet.

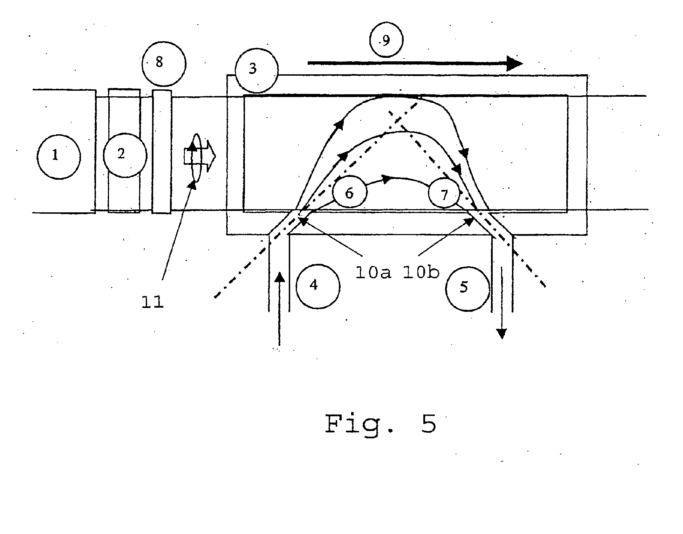

Ein besonders einfaches Design für die optische Pumpzelle mit, im Vergleich zum Stand der Technik, verminderter Wandrelaxation ist in Fig. 5 dargestellt. Diese eignet sich z.B. zur Produktion von hyperpolarisiertem Xenon und anderen Edelgasen. Gegenüber der Standardpumpzelle sind die Ein- und Auslassstutzen 10a und 10b anstelle eines 90° Winkels um definierte Winkel (6,7) geneigt. Ziel ist es die Rb-Bedampfung des Eintrittsfensters für den Laser zu minimieren und die Wechselwirkung des Stroms des Prozessgases zu vermindern, sowie Berührungen mit der Innenwand der Zelle im Vergleich zum Stand der Technik zu verringern.

Realisiert wurde eine Zelle mit Ein- und Auslass, welcher zur Längsseite der zylindrischen Probenzelle einen Winkel von 45° aufweist. Die Messungen der absoluten Xe-Polarisation zeigen, dass mit diesem Zelldesign Xe-Polarisationen in der Zelle von ca. 50 % (gemessen wurden ca. 40 % Polarisation hinter einem 7 m langen PFA-Schlauch mit einem Innendurchmesser von 1,5 mm) erreicht werden.

Eine weitere Steigerung der Xe-Polarisation ist durch Optimierung der Winkel der Ein- und Auslassstutzen sowie durch die Einstrahlung des Lasers entgegen der Strömungsrichtung möglich.

Fig. 5 zeigt im übrigen:

1 Rb Laser
2 Polarisationsoptik
3 Probenzelle
4 Eingang Prozessgas, bzw. Gemisch mit zu hyperpolarisierenden Atomen
5 Ausgang hyperpolarisiertes Xe
6 mittlerer Eintrittswinkel der Strömung in die Zelle
7 mittlerer Austrittswinkel der Strömung aus der Zelle
8 $\lambda$/4 Platte zur Erzeugung zirkular-polarisierter Lichter
9 Magnetfeld
10a, 10b Ein- und Auslassstutzen in die Probenzelle, hier um 45° zur Längsachse der Probenzelle geneigt

## Patentansprüche

1. Verfahren zur Hyperpolarisation von Atomkernen durch optisches Pumpen von Atomen in einem Gasgemisch in einer Probenzelle (5, 39, 55), wobei das Gemisch eine optisch pumpbare Spezies enthält, in welcher mittels Laserlicht eine Polarisation von Elektronenspins erreicht werden kann, sowie Atome mit den zu hyperpolarisierenden Atomkernen, und optional für die Hyperpolarisation inerte Verbindungen, wie z. B. Puffer- und Quenchgase,
**dadurch gekennzeichnet,**
**dass** das Gemisch derartig mit Druck beaufschlagt bis zu mindestens einem Absaugrohr in der Probenzelle geleitet wird, dass die Verweilzeit der hyperpolarisierten Kerne in der Probenzelle geringer ist als deren Diffusionszeit zu den Innenwänden der Probenzelle, wobei

- die Probenzelle über zwei gegenüberliegende Wände verfügt und das Gasgemisch mittig als freier Strahl durch eine der Wände in die Probenzelle injiziert wird und sich ohne Wandkontakt bis zur gegenüberliegenden Wand ausbreitet und dort mittig durch das Absaugrohr abgeführt wird; oder
- eine zylindrische Probenzelle verwendet wird, welche einen Ein- und einen Auslassstutzen (4, 5) aufweist, welche axial beabstandet sind und beide Stutzen aufeinander zugeneigt sind, wobei sowohl der einfließende Gasstrom als auch der ausfließende Gasstrom mit einem Vektor, der parallel zur Längsachse der Probenzelle verläuft und vom Einlass- zum Auslassstutzen zeigt, einen Winkel von 45° einschließt; oder
- die Zelle zylindrisch ist und über eine Düse verfügt, und das Gemisch in axialer Richtung mittig in die Probenzelle geleitet wird, wobei mehrere Absaugrohre (4) radial zur Probenzelle angeordnet sind und sich im gleichen Abstand von der Düse befinden, und durch diese Absaugrohre das Gasgemisch abgesaugt wird; oder
- ein Mantelstrom (38) aus einer inerten Verbindung zur Abtrennung des Gemisches von den Innenwänden in die Probenzelle geleitet wird.

2. Verfahren nach Anspruch 1,
**dadurch gekennzeichnet,**
**dass** das Gemisch an der Stelle ausgekoppelt wird, an der die Intensität des Laserlichts am größten ist.

3. Verfahren nach einem der vorhergehenden Ansprüche,
**dadurch gekennzeichnet,**
**dass** die Wände der Probenzelle gekühlt werden.

4. Verfahren nach einem der vorhergehenden Ansprüche,
**dadurch gekennzeichnet,**
**dass** der Spinaustausch indirekt über eine nicht optisch pumpbare Spezies auf den Kernspin eines zu hyperpolarisierenden Kerns erfolgt.

5. Verfahren nach einem der vorhergehenden Ansprüche,
**dadurch gekennzeichnet,**
**dass** $^{129}Xe$, $^{3}He$ oder $^{13}CO_2$ hyperpolarisiert wird.

6. Verfahren nach einem der vorhergehenden Ansprüche,

**dadurch gekennzeichnet,**
**dass** das Laserlicht senkrecht zur Strömungsrichtung des in der Probenzelle fließenden Gemisches in die Probenzelle eingestrahlt wird.

**7.** Verfahren nach einem der vorhergehenden Ansprüche,
**dadurch gekennzeichnet,**
**dass** das Laserlicht im Gegenstrom zur Strömungsrichtung des in der Probenzelle fließenden Gemisches in die Probenzelle eingestrahlt wird.

**8.** Vorrichtung zur Durchführung des Verfahrens nach einem der Patentansprüche 1-7, umfassend eine Probenzelle (5, 39, 55), sowie damit in Verbindung stehende Leitungen Leitungen und Vorratsbehälter für die optisch pumpbare Spezies, die Atome mit den zu hyperpolarisierenden Atomkernen und gegebenenfalls für die Hyperpolarisation inerte Verbindungen, und einen Laser zum optischen Pumpen, wobei entweder

- die Probenzelle zylinderförmig ist und einen Ein- und einen Auslassstutzen (4,5) aufweist, welche axial beabstandet sind und beide Stutzen aufeinander zugeneigt sind, so dass sowohl der einfließende Gasstrom als auch der ausfließende Gasstrom mit einem Vektor, der parallel zur Längsachse der Probenzelle verläuft und vom Einlass- zum Auslassstutzen zeigt, einen Winkel von 45° einschließt; oder
- die Probenzelle zylinderförmig ist und über eine Düse verfügt, um das Gemisch in axialer Richtung mittig in die Probenzelle zu leiten, wobei mehrere Absaugrohre (4) radial zur Probenzelle angeordnet und sich im gleichen axialen Abstand von der Düse befinden, um das Gasgemisch abzusaugen; oder
- die Vorrichtung über eine zusätzliche Gasversorgung (31c) verfügt, zur Bereitstellung eines inerten Gases, und die Probenzelle über zwei Grundflächen und eine diese verbindende Mantelfläche verfügt, wobei in einer der Grundflächen mittig eine Einlassöffnung vorgesehen ist für die Einleitung des Gasgemisches und in dieser Grundfläche oberhalb und unterhalb der mittigen Einlassöffnung jeweils ein weiterer Gaseinlass vorgesehen sind, welche mit der zusätzlichen Gasversorgung verbunden sind, um ein inertes Gas in die Probenzelle zu leiten zur Ausbildung eines Mantelstroms (38), der das mittig eingeleitete Gasgemisch von den Innenwänden der Probenzelle abtrennt.

**9.** Vorrichtung nach Anspruch 8,
**dadurch gekennzeichnet,**
**dass** im Falle mehrerer radial zur Probenzelle angeordneter Absaugrohre mindestens ein Laser (8) derartig angeordnet ist, dass das Laserlicht senkrecht und/oder im Gegenstrom zur Flussrichtung des Gemisches in die Probenzelle eingestrahlt wird.

**10.** Vorrichtung nach einem der vorhergehenden Ansprüche 8 bis 9,
**dadurch gekennzeichnet, dass** die Probenzelle mindestens ein Eintrittsfenster für das Laserlicht aufweist, das im größtmöglichen Abstand zum Eingang der Probenzelle für die optisch pumpbare Spezies angeordnet ist.

**11.** Vorrichtung nach einem der vorhergehenden Ansprüche 8 bis 10,
**gekennzeichnet durch**
Mittel zur Kühlung der Probenzellwände.

## Claims

**1.** Method for the hyperpolarisation of atomic nuclei by means of optical pumping of atoms in a gas mixture in a sample cell (5, 39, 55), wherein the mixture comprises an optically pumpable species in which polarisation of the electron spin can be achieved by means of laser light, and also comprises atoms with the atomic nuclei to be hyperpolarised, and optionally comprises compounds for hyperpolarisation which are inert, for example, buffer and quench gases, **characterised in that** pressure is applied to the mixture and said mixture is fed to at least one suction pipe in the sample cell, such that the dwell time of the hyperpolarised nuclei in the sample cell is shorter than the diffusion time thereof to the inner walls of the sample cell, wherein

- the sample cell has two mutually opposing walls and the gas mixture is injected centrally into the sample cell as a free jet through one of the walls and spreads out without making contact with the wall, as far the opposing wall and there is conducted away centrally through the suction pipe; or
- a cylindrical sample cell is used which comprises an inlet port and an outlet port (4, 5) which are spaced apart axially and are both angled toward one another, wherein both the inflowing gas stream and also the outflowing gas stream includes an angle of 45° with a vector which is aligned parallel to the longitudinal axis of the sample cell and points from the inlet port to the outlet port; or

- the cell is cylindrical and comprises a nozzle and the mixture is conducted centrally in the axial direction into the sample cell, wherein a plurality of suction pipes (4) are arranged radially to the sample cell and are located at the same separation from the nozzle, and the gas mixture is drawn out through said suction pipes; or

- a by-pass flow (38) of an inert compound is fed from the inner walls to the sample cell to separate the mixture from the inner walls.

2. Method according to claim 1, **characterised in that** the mixture is decoupled at the site where the intensity of the laser light is greatest.

3. Method according to one of the preceding claims, **characterised in that** the walls of the sample cell are cooled.

4. Method according to one of the preceding claims, **characterised in that** the spin exchange takes place indirectly via a non-optically pumpable species to the nuclear spin of a nucleus to be hyperpolarised.

5. Method according to one of the preceding claims, **characterised in that** $^{129}Xe$, $^{3}He$ or $^{13}CO_2$ is hyperpolarised.

6. Method according to one of the preceding claims, **characterised in that** the laser light is radiated into the sample cell perpendicular to the direction of flow of the mixture flowing in the sample cell.

7. Method according to one of the preceding claims, **characterised in that** the laser light is radiated into the sample cell contrary to the direction of flow of the mixture flowing in the sample cell.

8. Device for carrying out the method according to one of the claims 1-7, comprising a sample cell (5, 39, 55) and pipes and supply containers connected thereto for the optically pumpable species, also comprising the atoms having the nuclei to be hyperpolarised and optionally for the compounds for hyperpolarisation which are inert, and a laser for optical pumping, wherein either

- the sample cell is configured cylindrical and comprises an inlet port and an outlet port (4, 5) which are spaced apart axially and are both angled toward one another, so that both the inflowing gas stream and also the outflowing gas stream includes an angle of 45° with a vector which is aligned parallel to the longitudinal axis of the sample cell and points from the inlet port to the outlet port; or

- the sample cell is cylindrical and comprises a nozzle in order to conduct the mixture centrally in the axial direction into the sample cell, wherein a plurality of suction pipes (4) are arranged radially to the sample cell and are located at the same separation from the nozzle in order to draw out the gas mixture; or

- the device has an additional gas supply (31c) for providing an inert gas and the sample cell has two base surfaces and a jacket surface joining said base surfaces, wherein an inlet opening is centrally provided in one of the base surfaces for introducing the gas mixture, and a further gas inlet is provided in said base surface above and beneath the central inlet opening, in each case, being connected to the further gas supply in order to conduct an inert gas into the sample cell to form a by-pass flow (38) which separates the centrally introduced gas mixture from the interior walls of the sample cell.

9. Device according to claim 8, **characterised in that** in the case of a plurality of suction pipes arranged radially to the sample cell, at least one laser (8) is arranged such that the laser light is radiated into the sample cell perpendicular and/or in counterflow to the direction of flow of the mixture.

10. Device according to one of the preceding claims 8 to 9, **characterised in that** the sample cell has at least one entry window for the laser light, said window being arranged at the greatest possible distance from the entry of the sample cell for the optically pumpable species.

11. Device according to one of the preceding claims 8 to 10, **characterised by** means for cooling the sample cell walls.

**Revendications**

1. Procédé en vue de l'hyperpolarisation de noyaux atomiques par pompage optique d'atomes dans un mélange gazeux dans une cellule échantillon (5, 39, 55), sachant que le mélange gazeux contient une espèce optiquement pompable, dans laquelle on peut obtenir une polarisation du spin électronique à l'aide de lumière laser, ainsi que des atomes avec des noyaux atomiques hyperpolarisants, et, en option pour l'hyperpolarisation, des composés inertes, comme par exemple des gaz tampon et des gaz de trempe,

**caractérisé en ce**

**que** le mélange mis sous pression de cette façon est dirigé dans la cellule échantillon jusqu'à au moins un tube d'aspiration, que le temps de résidence des noyaux hyperpolarisants dans la cellule échantillon est inférieur à leur temps de diffusion en direction des parois internes de la cellule échantillon, sachant

- **que** la cellule échantillon dispose de deux parois opposées et le mélange gazeux est injecté dans la cellule échantillon au milieu en tant que jet libre à travers une des parois et se répartit sans mise en contact avec les parois jusqu'à la paroi opposée et y est évacué au milieu par le tube d'aspiration ; ou
- **que** l'on utilise une cellule échantillon cylindrique, qui présente des tubulures d'entrée et de sortie (4, 5) qui sont écartées axialement l'une de l'autre et qui sont inclinées l'une sur l'autre, sachant que non seulement le courant gazeux affluant mais aussi le courant gazeux sortant font un angle de 45° avec un vecteur, qui est aligné parallèlement à l'axe longitudinal de la cellule échantillon et qui s'étend de la tubulure d'entrée jusqu'à la tubulure de sortie ; ou
- **que** la cellule est cylindrique et dispose d'une buse, et le mélange est dirigé au milieu dans la cellule échantillon en direction axiale, sachant que plusieurs tubes d'aspiration (4) sont disposés de manière radiale par rapport à la cellule échantillon et se trouvent à la même distance de la buse, et que le mélange gazeux est aspiré à travers ces tubes d'aspiration ; ou
- **qu'**un courant enveloppe (38), constitué d'un composé inerte, est dirigé dans la cellule échantillon en vue de la séparation du mélange vis-à-vis des parois internes.

**2.** Procédé selon la revendication 1,
**caractérisé en ce**
**que** le mélange est découplé à l'endroit où l'intensité de la lumière laser est la plus forte.

**3.** Procédé selon l'une quelconque des revendications précédentes,
**caractérisé en ce**
**que** les parois de la cellule échantillon sont refroidies.

**4.** Procédé selon l'une quelconque des revendications précédentes,
**caractérisé en ce**
**que** l'échange de spin se fait indirectement par l'intermédiaire d'une espèce non optiquement pompable sur le spin nucléaire d'un noyau hyperpolarisant.

**5.** Procédé selon l'une quelconque des revendications précédentes,
**caractérisé en ce**
**que** $^{129}Xe$, $^{3}He$ ou $^{13}CO_2$ est hyperpolarisé.

**6.** Procédé selon l'une quelconque des revendications précédentes,
**caractérisé en ce**
**que** la lumière laser est irradiée dans la cellule échantillon perpendiculairement à la direction d'écoulement du mélange affluant dans celle-ci.

**7.** Procédé selon l'une quelconque des revendications précédentes,
**caractérisé en ce**
**que** la lumière laser est irradiée dans la cellule échantillon à contre-courant de la direction d'écoulement du mélange affluant dans celle-ci.

**8.** Dispositif en vue de l'exécution du procédé selon l'une des revendications 1 à 7, comportant une cellule échantillon (5, 39, 55), ainsi que les conduites et réservoirs raccordés pour l'espèce optiquement pompable, les atomes avec les noyaux atomiques hyperpolarisants et, le cas échéant, en vue de l'hyperpolarisation, des composés inertes, et un laser destiné au pompage optique, sachant

- que la cellule échantillon est cylindrique et présente des tubulures d'entrée et de sortie (4, 5) qui sont écartées l'une de l'autre et qui sont inclinées l'une sur l'autre, de telle sorte que non seulement le courant gazeux affluant mais aussi le courant gazeux sortant fassent un angle de 45° avec un vecteur, qui est aligné parallèlement à l'axe longitudinal de la cellule échantillon et qui s'étend de la tubulure d'entrée jusqu'à la tubulure de sortie ; ou
- que la cellule échantillon est cylindrique et dispose d'une buse, pour diriger le mélange dans la cellule échantillon au milieu en direction axiale, sachant que plusieurs tubes d'aspiration (4) sont disposés radialement par rapport à la cellule échantillon et se trouvent à la même distance axiale de la buse, pour évacuer par aspiration le mélange gazeux ; ou
- que le dispositif dispose d'une alimentation gazeuse supplémentaire (31c) en vue de la mise à disposition d'un gaz inerte et la cellule échantillon dispose de deux surfaces de base et d'une surface enveloppe reliant celles-ci, sachant que, dans une des surfaces de base, est prévu au milieu un orifice d'entrée pour l'introduction du mélange gazeux et que l'on prévoit dans cette surface de base, au-dessus et en-dessous de l'orifice d'entrée central, à chaque fois des admissions de gaz supplémentaires, qui sont reliées à l'alimentation de gaz supplémentaire, pour diriger un gaz inerte dans la cellule échantillon en vue de la formation d'un courant enveloppe (38), qui sépare le mélange gazeux introduit au milieu des parois internes de la cellule échantillon.

9.  Dispositif selon la revendication 8,
    **caractérisé en ce**
    **que** l'on dispose, dans le cas de plusieurs tubes d'aspiration disposés radialement par rapport à la cellule échantillon, d'au moins un laser (8) de façon à ce que la lumière laser soit irradiée dans la cellule échantillon perpendiculairement par rapport à et/ou à contre-courant de la direction d'écoulement du mélange gazeux.

10. Dispositif selon l'une quelconque des revendications précédentes 8 à 9,
    **caractérisé en ce que** la cellule échantillon présente au moins une fenêtre d'entrée pour la lumière laser, qui est disposée pour l'espèce optiquement pompable à la distance la plus grande possible de l'entrée de la cellule échantillon.

11. Dispositif selon l'une quelconque des revendications précédentes 8 à 10,
    **caractérisé par**
    des moyens en vue du refroidissement des parois de la cellule échantillon.

Fig. 1

Fig. 2

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5860295 A
- WO 9908766 A

- US 20020107439 A1

**In der Beschreibung aufgeführte Nicht-Patentliteratur**

- **HAPPER et al.** *Phys. Rev. A,* 1984, vol. 29, 3092
- **DRUCKSCHRIFT DRIEHUYS et al.** *Appl. Phys. Lett.,* 1996, vol. 69, 1668

- **S. APPELT ; A. BEN-AMAR BARANGA ; C. J. ERICKSON ; M. V. ROMALIS ; A. R. YOUNG ; W. HAPPER.** *Phys. Rev. A,* 1998, vol. 58, 1412